# EUROPEAN PATENT SPECIFICATION

(11) **EP 1 304 250 B1**
(45) Date of publication and mention of the grant of the patent: **30.08.2006**
(21) Application number: 02256904.0
(22) Date of filing: 03.10.2002
(51) Int. Cl.: B60K 28/02, G08B 21/06

(54) **Biological condition measurement apparatus and method, mobile unit navigation system and method, library apparatus, and computer program**
Gerät zum Messen eines biologischen Zustandes und Verfahren, mobile Navigationssystemeinheit und Verfahren, Datenbibliotheksgerät und Computerprogramm
Appareil de mesure de condition biologique et procédé, unité de système de navigation mobile et procédé, appareil de bibliothèque de données et logiciel d'ordinateur

(30) Priority: 12.10.2001 JP 2001315229
(43) Date of publication of application: 23.04.2003
(73) Proprietor: Pioneer Corporation, Meguro-ku, Tokyo (JP)
(72) Inventor: Yanagidaira, Masatoshi Pioneer Corp., Res. and Dev, Tsurugashima-shi, Saitama (JP); Yasushi, Mitsuo Pioneer Corp., Res. and Dev. Lab., Tsurugashima-shi, Saitama (JP)
(74) Representative: Haley, Stephen

(56) References cited:
- DE-A- 4 338 244
- DE-A- 4 407 935
- DE-A- 19 918 645
- US-A- 6 104 296
- PATENT ABSTRACTS OF JAPAN vol. 2000, no. 12, 3 January 2001 (2001-01-03) & JP 2000 262481 A (PIONEER ELECTRONIC CORP), 26 September 2000 (2000-09-26)

## Description

This invention relates to technical fields of a biological condition measurement apparatus and method for detecting the biological condition of the operator of a mobile unit and providing the convenience of mobile unit operation, etc., a mobile unit navigation system using the biological condition measurement apparatus, a mobile unit navigation method using the biological condition measurement method, a library apparatus using the biological condition measurement apparatus, and a biological condition measurement computer program.

In recent years, research and development of electronic control for controlling running of an automobile and a navigation system for aiding in driving have become widespread markedly.

As an example of electronic control of running of an automobile, ACC is available. It automatically detects a preceding vehicle by a millimeter wave radar, controls accelerator and a brake, maintains the vehicle speed and the distance between vehicles, and decelerates or stops the vehicle matching motion of the preceding vehicle on a traffic jam road. Some vehicles have a function of producing a warning sound or displaying a warning if the distance between vehicles becomes shorter than a predetermined distance. That is, the ACC function determines the running state of the preceding vehicle and causes the vehicle to perform automatic running relative to the vehicle speed and the distance between vehicles, thereby lightening the operation load on the driver. If the vehicle is not preceded by any vehicle, the driver can be allowed to drive at comfortable run: speed set by the driver.

A navigation system for aiding in driving displays map data, current position data, route data to the destination, various guide data, etc., on a display or outputs a navigation guide message, warning message, etc., by voice through a loudspeaker installed in the vehicle. The current position data is provided by self-contained position measurement units of a speed sensor, an acceleration sensor, an angular speed sensor, etc., for providing position information, installed in the vehicle (namely, various position measurement units for traditional self-contained navigation (SCN)) or GPS (Global Positioning System). Further, a communication navigation system being developed at present has radio communicator and communicates with external information sources and catches and displays objective information or outputs the objective information by voice for aiding in driving.

In addition to the above-described apparatus, etc., a system for detecting the biological condition of the driver driving a vehicle and controlling the operation of the vehicle in response to biological information or informing the driver of the condition for attracting the attention of the driver is proposed. For example, JP-A-6-255520 discloses a system which comprises a steering wheel provided with a unit for detecting the heart rate of the driver to detect the biological condition of the driver and determines whether or not the driver is irritated from the heart rate and controls the vehicle. The system inputs the biological condition of the driver and the behavior condition of the vehicle and produces control output of the vehicle from the analysis result of the biological. condition and the behavior condition. As a method of measuring the heart rate, a process for determining that the vehicle is in a stable state is provided to avoid noise mixing caused by vibration, etc., of the vehicle, and the heart rate measured in the time period in which the vehicle is in a stable state is adopted as the effective heart rate.

However, in the method of detecting the heart rate, the heart rate is measured only under the condition that the vehicle behavior is stable and thus is not necessarily measured in the time period in which the biological condition of the driver is stable. That is, the time period until the biological condition becomes stable from reception of change in the vehicle behaviour generally differs from the stable time period of the vehicle and is an ample time. That is, the heart rate just after the vehicle behaviour becomes stable is not the heart rate measured when the driver is in a stable biological condition, and if the biological condition of the driver is determined based on the heart rate just after the vehicle behaviour becomes stable, there is a fear of giving an erroneous vehicle operation instruction.

DE-A-4338244 discloses a warning system for a vehicle, having a monitoring system for the vehicle, environment and driver which takes steps to avoid danger if the driver is unaware, according to the preamble of claim 1.

It is therefore desirable to provide a biological condition measurement apparatus and method capable of measuring the biological information of the heart rate, etc., in a stable state, a mobile unit navigation system and method for aiding in stable vehicle operation based on the biological condition measurement result of the biological condition measurement apparatus and method, a library apparatus for classifying and editing various types of content of music, pictures, etc., based on the biological condition measurement result of the biological condition measurement apparatus and method, and a computer program for measuring the biological information of the heart rate, etc., in a stable state.

According to a first aspect of the invention, there is provided a biological condition measurement apparatus comprising:
a behaviour condition detector which detects the behaviour condition of a mobile unit;
a mobile unit stable state determining device which determines whether or not said mobile unit is in a stable state based on the behaviour condition detected by said behaviour condition detector;
a biological condition detector which detects the biological condition of an operator operating said mobile unit;
an operator stable state determining device which determines whether or not the operator is in a stable state based on the biological condition detected by said biological condition detector; and
a processor which adopts biological information indicating the biological condition detected by said biological condition detector as valid biological information, and executes at least one of analysis processing, record processing, and output processing of the biological information, if said mobile unit stable state determining device determines that said mobile unit is in a stable state and said operator stable state determining device determines that the operator is in a stable state,
characterised in that said processor starts processing of the biological information after the expiration of a predetermined time T₀ since said behaviour condition detector detects a change in the behaviour condition of said mobile unit.

According to the biological condition measurement apparatus of the invention, at the operating time of the apparatus, the behaviour condition detector detects the behaviour condition of the mobile unit and the mobile unit stable state determining device determines whether or not the mobile unit is in a stable state based on the detection result. In parallel with this, the biological condition detector detects the biological condition of the operator of the mobile unit, and the operator stable state determining device determines whether or not the operator is in a stable state based on the detection result. If it is determined that the behavior condition of the mobile unit is in a stable state and that the biological condition of the operator is in a stable state, the processor adopts the biological information indicating the biological condition detected by the biological condition detector as valid biological information and executes at least one of analysis processing, record processing, and output processing of the biological information.

Therefore, it is made possible to analyze, record, or output the biological information indicating the biological condition of the operator of the mobile unit in the time period in which the mobile unit is in a stable state and the biological condition of the operator is also in a stable state; the biological condition can be measured with exceptionally high accuracy as compared with the related art of measuring the biological condition of the operator based only on the stable state of the mobile unit.

Particularly, according to the research of the present inventors, it takes time in stabilizing the biological condition accompanying behavior fluctuation of the mobile unit. For example, assuming a vehicle as the mobile unit, it takes about several seconds in stable state transition of the vehicle, but it takes about several ten seconds in stabilizing the biological condition. Therefore, generally, in measurement just after the mobile unit becomes stable, the effect caused by condition change of the mobile unit is left in the biological condition and it is difficult to measure the correct biological condition. However, the biological condition measurement apparatus of the invention can solve this problem as described above.

In the biological condition measurement apparatus of the invention, the processor starts processing of the biological information after the expiration of predetermined time To since the behaviour condition detector detected change in the behaviour condition of the mobile unit. In this regard, it takes a long time in stabilizing the biological condition based on change in the behaviour condition of the mobile unit relative to the time taken for the mobile unit to become the stable state after the behaviour condition of the mobile unit changes as described above. Thus, to start processing of the biological information, the predetermined time To for the biological condition to become stable is preset and actual measurement is started at the time. Accordingly, it is made possible to measure the biological condition more precisely and the processing load on the CPU, etc., is decreased.

The biological information indicating the biological condition thus detected in the stable state is validated and the processor performs analysis processing, record processing, or output processing and it is made possible to precisely keep track of the biological condition of the operator from the processing result. If information of control, etc., of the mobile unit, for example, is created based on the processing results it is made possible to control the mobile unit safely and preferably.

"Measurement of the biological condition" or "measuring the biological condition" mentioned in the specification means that at least one of analysis processing, record processing, or output processing of the biological information indicating the biological condition detected by the biological condition detector is performed.

In one mode of the biological condition measurement apparatus of the invention, if fluctuation of the behavior condition detected by the behavior condition detector is within a predetermined range, the mobile unit stable state determining member determines that the mobile unit is in a stable state.

According to the mode, it is considered that if the mobile unit is in the stable state, the detection output of the behavior condition detector contains a slight fluctuation component, and also in this case, the mobile unit is determined to be in the stable state and the biological condition can be measured. As the factors of fluctuation, slight speed change of the mobile unit, mobile unit fluctuation affected by the road surface condition, electric noise, and the like are assumed. Thus, considering that if the allowable range of the output fluctuation is set narrow, the operation does not become stable and on the other hand, if the allowable range is set wide, consequently, measurement of the biological condition with high accuracy is made impossible, preferably the allowable range is determined separately and specifically in view of the environment in which the biological conditionmeasurement apparatus is actuallyused and the specifications of the apparatus.

The allowable range of the output fluctuation is not limited to the fixing method and may be allowed to be variably set in the optimum range in response to the characteristic of the mobile unit, the learning effect, etc.

In another mode of the biological condition measurement apparatus of the invention, if fluctuation of the biological condition detected by the biological condition detector is within a predetermined range, the operator stable state determining device determines that the operator is in a stable state.

According to the mode, it is considered that if the operator is in the stable state, the detection output of the biological condition detector contains a slight fluctuation component, and also in this case, the operator is determined to be in the stable state and the biological condition can be measured. As the factors of fluctuation, a slight body move of the operator, acoustic disturbance, electric noise, and the like are assumed. Thus, considering that if the allowable range of the output fluctuation is set narrow, the operation does not become stable and on the other hand, if the allowable range is set wide, consequently, measurement of the biological condition with high accuracy is made impossible, preferably the allowable range is determined separately and specifically in view of the environment in which the biological condition measurement apparatus is actually used and the specifications of the apparatus.

The allowable range of the output fluctuation is not limited to the fixing method and may be allowed to be variably set in the optimum range in response to the characteristic of the operator, the learning effect, etc.

The predetermined time To may be allowed to be set arbitrarily.

In doing so, it is made possible to set the measurement start timing of the biological condition, namely, the processing start timing of the biological information to the optimum timing in response to the operator's individual difference. According to the research of the inventors, the time taken in stabilizing the biological condition varies from one operator to another and it is also assumed that the time can be shortened as the learning effect. Therefore, it is desired that the optimum predetermined time To matching the condition of each operator individually can be set.

The processor may perform processing of the biological information at predetermined time tₛ after the expiration of the predetermined time To.

In doing so, processing of the biological information is performed at the time tₛ after the expiration of the predetermined time To after the condition change of the mobile unit is made. The time tₛ is the measurement time unit in detection processing, record processing, or output processing of the biological information and examination of the validity of the biological information and further preparation of mobile unit control information, etc., at the later stage based on the biological information are performed based on the biological condition measured in the unit time. Several units may be used in a comprehensive manner to cover processing at the later stage.

In the mode involving the predetermined time tₛ, when the processor performs processing of the biological information at the predetermined time tₛ, if the mobile unit stable state determining device determines that the behaviour of the mobile unit within analysis time is in a stable state, the processor may validate the biological information and on the other hand, if the mobile unit stable state determining device does not determine that the behaviour of the mobile unit is in a stable state, the processor may again perform processing of the individually can be set.

In the mode involving the predetermined time To, the processor performs processing of the biological information at predetermined time tₛ after the expiration of the predetermined time T₀.

In doing so, processing of the biological information is performed at the time tₛ after the expiration of the predetermined time T₀ after the condition change of the mobile unit is made. The time tₛ is the measurement time unit in detection processing, record processing, or output processing of the biological information and examination of the validity of the biological information and further preparation of mobile unit control information, etc., at the later stage based on the biological information are performed based on the biological condition measured in the unit time. Several units maybe used in a comprehensive manner to cover processing at the later stage.

In the mode involving the predetermined time tₛ, when the processor performs processing of the biological information at the predetermined time tₛ, if the mobile unit stable state determining member determines that the behavior of the mobile unit within analysis time is in a stable state, the processor may validate the biological information and on the other hand, if the mobile unit stable state determining device does not determine that the behavior of the mobile unit is in a stable state, the processor may again perform processing of the biological information at the predetermined time tₛ.

In doing so, to determine the validity of the biological information indicating the biological condition measured at the predetermined time tₛ, the stable state of the mobile unit at the predetermined time tₛ is determined. The mobile unit stable state determining member determines the stable state of the mobile unit, for example, in response to whether or not the detection result output of the behavior condition detector is within a predetermined range. If the detection result output is within the predetermined range, the biological information is validated and measurement of the biological condition is terminated. On the other hand, if the detection result outputs is out of the predetermined range, the biological information is invalidated, and again measurement is conducted at the time tₛ. That is, if the detection result output of the behavior condition detector is out of the predetermined range, it is determined that the behavior condition of the mobile unit is changed, and accordingly the biological condition is affected and the biological information does not become the biological information indicating the stable biological condition required.

In the mode involving the predetermined time tₛ, the predetermined time tₛ may be allowed to be set arbitrarily.

In doing so, it is made possible to set the predetermined time tₛ matching the operator's individual difference. It is also made possible to set the predetermined time tₛ considering the reaction speed of the mobile unit.

In the mode involving the predetermined time tₛ, the processor may perform processing of the biological information at two or more successive intervals of the predetermined time tₛ after the expiration of predetermined time To and may compare the processing results and if an error between the processing results is within a predetermined range, may validate the biological information.

In doing so, the validity of the biological information is determined based on more than one piece of the biological information provided by performing more than one processing, and more precise biological information can be provided. Therefore, it is made possible to generate a more precise and more safe mobile unit control signal:, etc., based on the biological information. Preferably, data provided by performing processing as large number of times within the load margin of the CPU and.the range of the time restriction is used.

In another mode of the biological condition measurement apparatus of the invention, the biological condition is physical and mental physiological conditions of the operator based on at least one of pulse, sweating, skin resistance, respiration, heart rate, and heart rate variability component.

According to the mode, it is made possible to detect the physical and mental physiological conditions of the operator comparatively easily, even if the operator is in the mobile unit or operates the mobile unit based on the pulses, sweating, skin resistance, respiration, heart rate, or heart rate variability component, or a combination of any two or more items.

According to a second aspect of the invention, there is provided a biological condition measurement method comprising:
a behaviour condition detection step of detecting the behaviour condition of a mobile unit;
a mobile unit stable state determination step of determining whether or not said mobile unit is in a stable state based on the behaviour condition detected in said behaviour condition detection step;
a biological condition detection step of detecting the biological condition of an operator operating said mobile unit;
an operator stable state determination step of determining whether or not the operator is in a stable state based on the biological condition detected in said biological condition detection step; and
a processing step of adopting biological information indicating the biological condition detected in said biological condition detection step as valid biological information, and executing at least one of analysis processing, record processing, and output processing of the biological information, if it is determined in said mobile unit stable state determination step that the mobile unit is in a stable state, and it is determined in said operator stable state determination step that the operator is in a stable state,
characterised by starting processing of the biological information after the expiration of a predetermined time To since detecting a change in the behaviour condition of the mobile unit.

According to the biological condition measurement method of the invention, as with the biological condition measurement apparatus of the invention described above, the behaviour condition of the mobile unit is detected and whether or not the mobile unit is in a stable state is determined based on the detection result. In parallel with this, the biological condition of the operator of the mobile unit is detected, and whether or not the operator is in a stable state is determined based on the detection result. If it is determined that the behaviour condition of the mobile unit is in a stable state and that the biological condition of the operator is in a stable state, the biological information indicating the biological condition detected is adopted as valid biological information and at least one of analysis processing, record processing, and output processing of the biological information is executed. Therefore, it is made possible to analyze, record, or output the biological information indicating the biological condition of the operator of the mobile unit in the time period in which the mobile unit is in a stable state and the biological condition of the operator is also in a stable state; the biological condition can be measured with exceptionally high accuracy as compared with the related art of measuring the biological condition of the operator based only on the stable state of the mobile unit.

According to a third aspect of the invention there is provided a mobile unit navigation system comprising:
a biological condition measurement apparatus according to the first aspect;
a mobile unit controller which applies control to the run state of said mobile unit based on the processing result of said processor;
a current position detector which detects the current position of said mobile unit; and
an information presenter which presents guide information for said mobile unit based on the detected current position.

According to the mobile unit navigation system of the invention, the above-described biological condition measurement apparatus of the invention is made integral with a general-purpose navigation system, whereby the biological information of the operator can be utilized effectively in the navigation system. Particularly, the mobile unit controller applies control to the run state of the mobile unit in response to the biological condition of the operator, whereby it is made possible to aid in operation of the mobile unit.

As the current position detector, a GPS receiver, a self-contained position measurement unit, etc., is available. As the information presenter, a liquid crystal display for displaying map information, an acoustic device for outputting guide information by voice, or the like is available.

One mode of the mobile unit navigation system of the invention further comprises a communicator which receives at least either of the guide information and source information of the guide information via an external communication network.

According to the mode, the communicator makes it possible to communicate with various communication sites via the external communication network, and advanced and various services can be received making the most of the biological information. Particularly, the mobile unit navigation system can be used as a communication navigation system terminal; the system site can be charged with map information of an enormous data amount and a map search involving a large CPU load, and it is made possible to realize a comparatively light-equipped mobile unit navigation system.

Another mode of the mobile unit navigation system of the invention further comprises run environment sensor which senses the run environment of the mobile unit; run state sensor which senses the run state of the mobile unit; and an ACC unit which controls running of the mobile unit basedon information provided for determination of operation of the mobile unit, sensed by the run environment sensor and the run state sensor.

According to the mode, the ACC unit makes it possible to operate the mobile unit in the optimum condition automatically based on the data of the run state, the run environment, and the biological condition of the mobile unit.

The ACC unit automatically detects a preceding vehicle by a millimeter wave radar, controls accelerator and a brake to maintain the vehicle speed and the distance between vehicles, and decelerates or stops the vehicle matching motion of the preceding vehicle on a traffic jam road. If the distance between vehicles becomes shorter than a predetermined distance, the ACC unit produces a warning sound, displays a warning, and decreases the driver's drive load for running the vehicle efficiently and with high safety. In addition to the basic functions, other functions of assisting driver's driving may be provided.

According to a fourth aspect of the invention, there is provided a mobile unit navigation method comprising:
a biological condition measurement method according to the second aspect;
a mobile unit control step of applying control to the run state of said mobile unit based on the processing result of said processing step;
a current position detection step of detecting the current position of said mobile unit; and
an information presenting step of presenting guide information for said mobile unit based on the detected current position.

According to the mobile unit navigation method of the invention, the above-described biological condition measurement method of the invention is made integral with a general-purpose navigation method, whereby the biological information of the operator can be utilized effectively in the navigation method. Particularly, the mobile unit control step is to apply control to the run state of the mobile unit in response to the biological condition of the operator, whereby it is made possible to aid in operation of the mobile unit.

One mode of the mobile unit navigation method of the invention further comprises a communication step of receiving at least either of the guide information and source information of the guide information via an external communication network.

According to the mode, the communication step makes it possible to communicate with various communication sites via the external communication network, and advanced and various services can be received making the most of the biological information. Particularly, the system site can be charged with map information of an enormous data amount and a map search involving a large CPU load, and it is made possible to realize a comparatively light-equipped mobile unit navigation method.

Another mode of the mobile unit navigation method of the invention further comprises: a run environment sense step of sensing the run environment of the mobile unit; a run state sense step of sensing the run state of the mobile unit; and a run control step of an ACC unit which controls running of the mobile unit based on information provided for determination of operation of the mobile unit, sensed in the run environment sense step and the run state sense step.

According to the mode, run control of the ACC unit is made possible based on the data of the run state, the run environment, and the biological condition of the mobile unit.

According to a fifth aspect of the invention, there is provided a library apparatus comprising:
a biological condition measurement apparatus according to the first aspect;
an outputting device which outputs content information including at least one of audio information, video information, and text information;
correlation analyzer which takes the correlation between the biological information processed by said processor and the content information when the content information is output; and
library creator which creates a library of the content information suiting taste of the operator based on the analysis result of said correlation analyzer.

According to the library apparatus of the invention, the correlation between the high-accuracy biological condition measured by the biological condition measurement apparatus and the content information as voice output or image output provided by the outputting member is analyzed. For example, when the operator listens to a specific musical piece, the biological condition of the operator is analyzed as to whether the operator becomes the biological condition indicating pleasure or unpleasure. Based on such an analysis result, it would be made possible to create a library suiting taste of the driver comparatively easily.

In one mode of the library apparatus of the invention, for the content information, feature information indicating the feature in the contents of the content information is preset, a library apparatus comprising the above-described biological condition measurement apparatus of the invention (containing various modes thereof) ; outputting device which outputs content information including at least one of audio information, video information, and text information; correlation analyzer which takes the correlation between the biological information processed by the processor and the content information when the content information is output; and library creator which creates a library of the content information suiting taste of the operator based on the analysis result of the correlation analyzer.

According to the library apparatus of the invention, the correlation between the high-accuracy biological conditions measured by the biological condition measurement apparatus and the content information as voice output or image output provided by the outputting member is analyzed. For example, when the operator listens to a specific musical piece, the biological condition of the operator is analyzed as to whether the operator becomes the biological condition indicating pleasure or unpleasure. Based on such an analysis result, it would be made possible to create a library suiting taste of the driver comparatively easily.

In one mode of the library apparatus of the invention, for the content information, feature information indicating the feature in the contents of the content information is preset, and the correlation analyzer takes the correlation between the preset feature information in place of the content information and the biological information.

According to the mode, feature information is preset for the content information. Since the correlation between the biological condition measured by the biological condition measurement apparatus and the feature information indicating the feature of the content information output by the outputting member is analyzed, it is made possible to create a library suiting taste of the driver comparatively easily based on such an analysis result.

An alternative mode of the library apparatus of the invention further comprises extractor which extracts..feature in the contents of the content information from the content information, wherein the correlation analyzer takes the correlation between feature information indicating the feature extracted by the extractor in place of the content information and the biological information.

According to the mode, feature is extracted from the content information and the feature information is related. Since the correlation between the biological conditionmeasured by the biological condition measurement apparatus and the feature information of the content information output by the outputting member is analyzed, it is made possible to create a library suiting taste of the driver comparatively easily based on such an analysis result.

According to a sixth aspect of the invention, there is provided a computer program for causing a computer to function as
a mobile unit stable state determining circuit which determines whether or not a mobile unit is in a stable state based on the behaviour condition of said mobile unit detected by a behaviour condition detector;
an operator stable state determining circuit which determines whether or not an operator operating said mobile unit is in a stable state based on the biological condition of the operator detected by a biological condition detector; and
a processor which adopts biological information indicating the biological condition detected by said biological condition detector as valid biological information, and executes at least one of analysis processing, record processing, and output processing of the biological information, if said mobile unit stable state determining circuit determines that said mobile unit is in a stable state and the operator stable state determining circuit determines that the operator is in a stable state,
characterised in that said program causes said computer, in functioning as said processor, to start processing of the biological information after the expiration of a predetermined time To since said behaviour condition detector detects a change in the behaviour condition of said mobile unit.

According to the computer program of the invention, the computer program is read from the record medium such as a CD (Compact Disc), a DVD, or a hard disk storing the computer program into a computer for execution. Alternatively, the computer program is downloaded into a computer through communicator and then is executed, whereby the biological condition measurement apparatus of the invention described above can be provided comparatively easily.

These and other functions and advantages of the invention will become more apparent as the description of preferred embodiments of the invention proceeds.

In the accompanying drawings:
FIG. 1 is a block diagram to show the configuration of a biological state measurement apparatus according to a first embodiment of the invention;
FIG. 2 is a characteristic diagram to show a biological information detection method of the biological state measurement apparatus according to the first embodiment of the invention;
FIG. 3 is a characteristic diagram to describe the stable state of a vehicle installing the biological state measurement apparatus according to the first embodiment of the invention;
FIG. 4 is a flowchart to show an operation flow of the biological state measurement apparatus according to the first embodiment of the invention;
FIG. 5 is a block diagram to show the configuration of a biological state measurement apparatus according to a second embodiment of the invention;
FIG. 6 is a block diagram to show the configuration of a biological condition sensor section of the biological state measurement apparatus according to the second embodiment of the invention;
FIG. 7 is a block diagram to show the configuration of a navigation system incorporating the biological state measurement apparatus according to the second embodiment of the invention;
FIG. 8 is a flowchart to show an operation flow of the second embodiment of the invention;
FIG. 9 is a block diagram to show the configuration of a library apparatus according to a third embodiment of the invention; and
FIG. 10 is a flowchart to show an operation flow of the third embodiment of the invention.

Referring now to the accompanying drawings, there are shown preferred embodiments of the invention. In the embodiments, a biological condition measurement apparatus of the invention is applied to a mobile unit. Here, it is assumed that the mobile unit is a vehicle.

### (First embodiment)

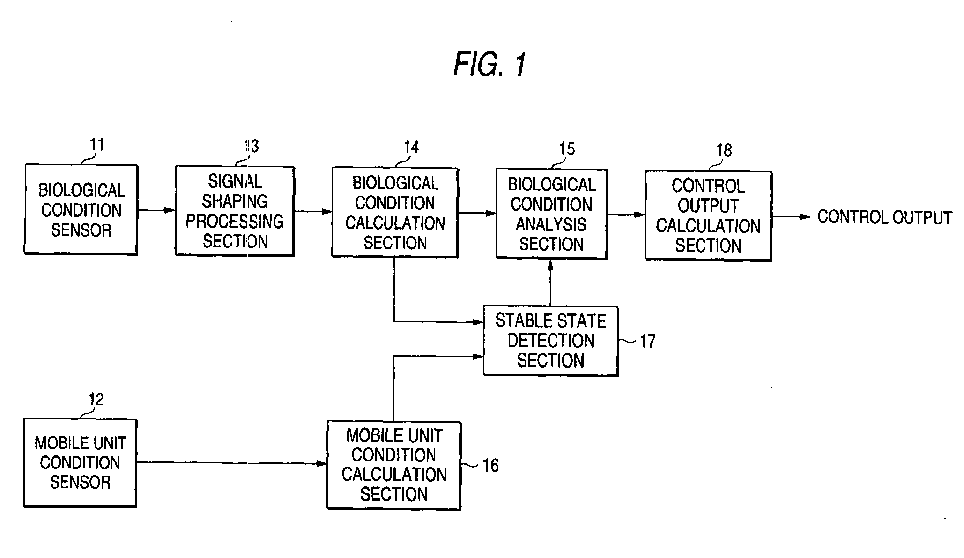

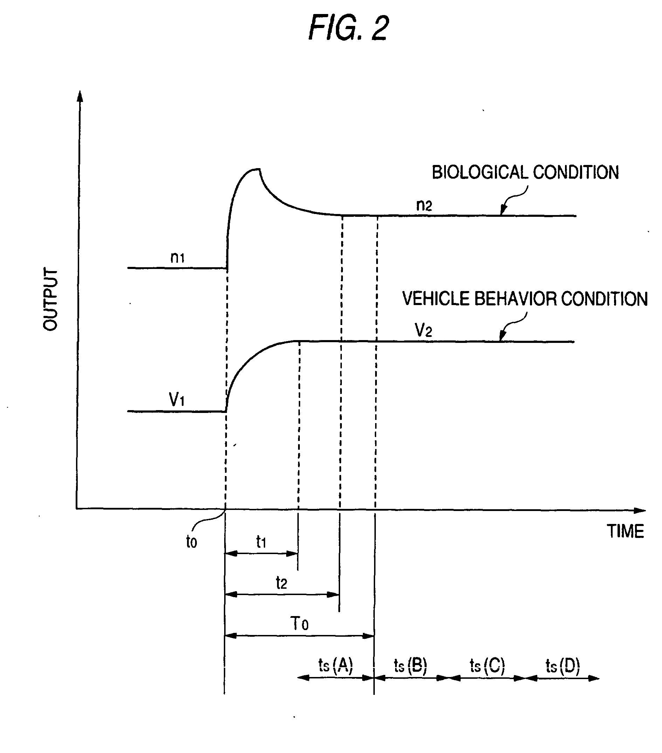

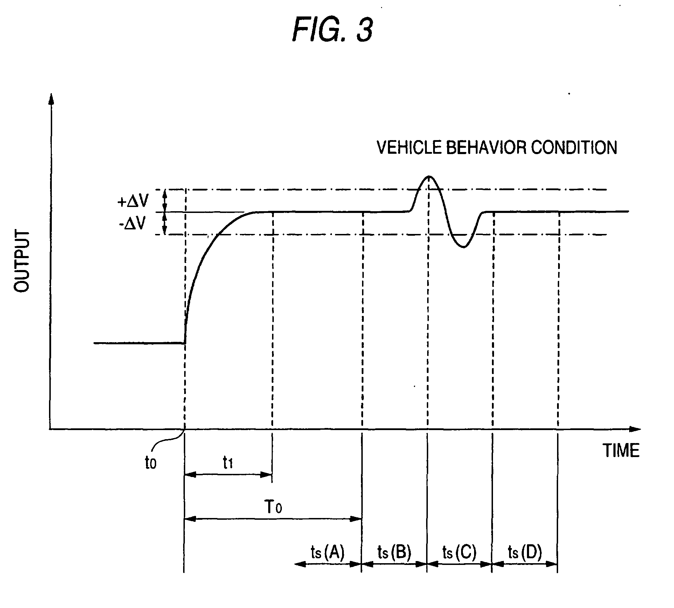

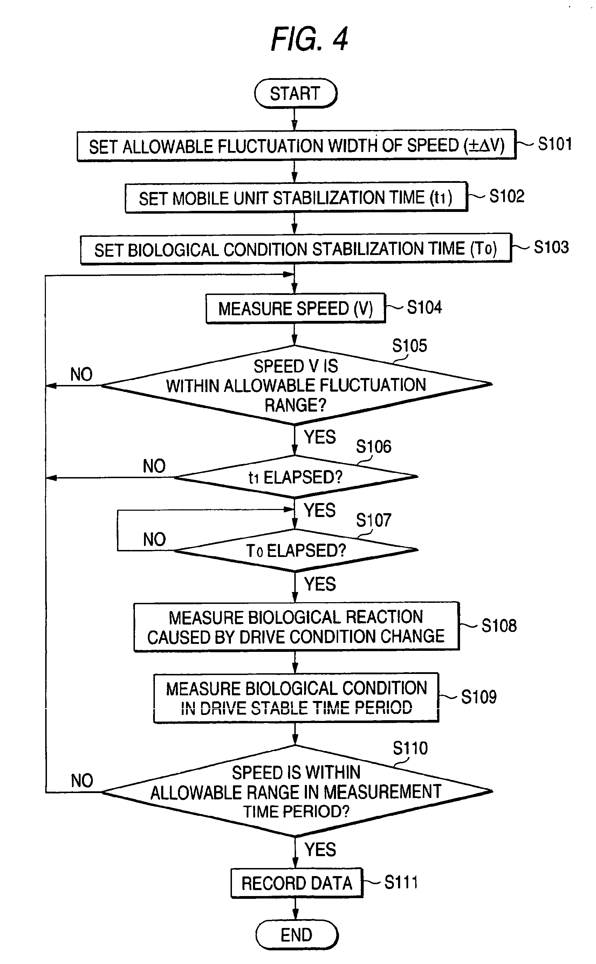

To begin with, a first embodiment will be discussed with reference to FIGS. 1 to 4. FIG. 1 is a block diagram to show the configuration of a biological condition measurement apparatus according to a first embodiment of the invention, FIG. 2 is a characteristic diagram to show a biological information detection method of the biological condition measurement apparatus, FIG. 3 is a characteristic diagram to describe the stable state of a vehicle installing the biological condition measurement apparatus, and FIG. 4 is a flowchart to show an operation flow of the biological condition measurement apparatus.

As shown in FIG. 1, the biological condition measurement apparatus comprises the blocks of a biological condition sensor 11, a mobile unit condition sensor 12, a signal shaping processing section 13, a biological condition calculation section 14, a biological condition analysis section 15, a mobile unit condition calculation section 16, a stable state detection section 17, and a control output calculation section 18.

The biological condition sensor 11 forms an example of biological condition detector which detects the mental or physical condition of the operator of the mobile unit; it detects pulses, sweating, skin resistance, respiration, heart rate, heart rate variability component, and the like. One or a combination of any two or more items is used to determine the physical and mental physiological conditions of the operator when the operator operates the mobile unit.

As the biological condition sensor 11, for example, to detect an electrocardiogram concerning pulses, for example, the potential difference between both hands can be detested by electrodes installed in a steering wheel section, a pulse wave to detect the peripheral blood amount in the hand put on the steering wheel by an optical sensor installed in the steering wheel section can be used, or a feeble microwave can be applied to the heart by a high-frequency circuit installed in a seatbelt, etc., and the phase difference between the applied signal and its return signal can be analyzed, thereby measuring the heart rate.

The heart rate of the driver rises in a condition in which the driver is strained or irritated, and change in the condition is determined from the electrocardiogram, whereby it is made possible to evaluate the mental condition of the driver. A method of analyzing spectrum change of fluctuation component of heart rate interval for evaluating the mental condition of the driver may be used. Further, the biological condition can also be known by checking the skin electric reaction (different from the electrocardiogram) between left and right hands or sweating based on skin electric resistance. The skin electric reaction indicating temporary strain is used in combination with another, whereby it is made possible to keep track of the biological condition of the operator still more accurately. In addition, other biological condition detection techniques than the techniques described above are used in the embodiment whenever necessary, of course.

The mobile unit condition sensor 12 forms an example of behavior condition detector which detects the behavior condition of the mobile unit. The main behavior condition is speed and a speed meter of the mobile unit can be used. Other behavior conditions include information of the mobile unit position, information of acceleration, information of mobile unit reaction, time information, for example, between the instant at which the operator operates the vehicle and the instant at which the mobile unit actually starts motion, and the like. The position of the mobile unit can be measured by a position measurement unit of GPS, etc., and as the acceleration, output of an acceleration sensor forming a self-contained position measurement unit can be used. As the time information of the mobile unit reaction, the time between the instant at which operation starts and the instant at which actually motion of the mobile unit starts for each type of operation can be measured according to a predetermined time count technique.

The signal shaping processing section 13 adjusts a detection signal from the biological condition sensor 11 to a signal form fitted for input to the following circuit, and is made up of a filter for extracting any desired signal, an amplifier for adjusting the signal level, etc., for example. It further comprises a sample-and-hold device, analog-digital converter, etc., required for performing digital signal processing.

The biological condition calculation section 14 quantitatively calculates the biological information of the operator of the mobile unit based on the biological information from the biological condition sensor 11. Quantitatively calculated are pulses, sweating, skin resistance, respiration, heart rate, heart rate variability component, and the like.

The biological condition analysis section 15 forms an example of processor which analyzes the mental or physical physiological condition of the operator based on a detection signal from the biological condition sensor 11. For example, if the heart rate rises, the driver is determined to be strained or irritated, or from sweating or change in the skin resistance, the driver is determined to be agitated or astonished. These conditions are presented qualitatively or quantitatively. The determination is made by comparing with a preset threshold value or a variable predetermined threshold value. Further, the biological condition may be determined by referring a preset table in response to a combination of two or more types of detection signals, or the biological condition may be inferred by an inference engine by referring to a knowledge base.

The mobile unit condition calculation section 16 quantitatively calculates the condition of the mobile unit based on speed information, of the mobile unit, position information of the mobile unit, information of acceleration, information of mobile unit reaction, etc., from the mobile unit condition sensor 12

The stable state detection section 17 forms an example of mobile unit stable state determining member and operator stable state determining member which determines whether or not the biological condition and the mobile unit condition reach the stable state based on the data found by the biological condition calculation section 14 and the mobile unit condition calculation section 16. If the stable state detection section 17 determines that both the biological condition and the mobile unit condition reach the stable state, it informs the biological condition analysis section 15 that the biological information detected by the biological condition sensor 11 is valid.

Particularly, to determine the condition of the mobile unit, whether or not the mobile unit condition reaches the stable state is determined after the behavior of the mobile unit changes. If output fluctuation indicating the behavior condition is within a predetermined range, it is determined that the mobile unit condition reaches the stable state; if the output fluctuation is outside the predetermined range, it is not determined that the mobile unit condition reaches the stable state.

On the other hand, to determine the condition of the operator, whether or not change in the biological condition of the operator accompanying behavior change of the mobile unit reaches the stable state is determined. If output fluctuation indicating the biological condition is within a predetermined range, it is determined that the condition reaches the stable state; if the output fluctuation is outside the predetermined range, it is not determined that the condition reaches the stable state.

The control output calculation section 18 forms and outputs control information to operate the vehicle based on the biological information indicating the biological condition in the stable state of the mobile unit behavior provided as described above. The control information may be displayed on a display for the driver or can also be used directly to control the run state of the mobile unit. For example, if the driver is irritated during vehicle driving, it is possible to present passing information and later leave operation to the driver. On the other hand, if it is determined that the vehicle (driver) is in a dangerous state, it is possible to perform operation for automatically stopping the vehicle. To present information for the driver, the display or an acoustic device is used.

Next, stability determination of mobile unit behavior and biological condition measurement timing will be discussed with reference to FIGS. 2 and 3.

The upper stage of FIG. 2 indicates the biological condition, for example, the heart rate and the lower stage represents the mobile unit behavior condition, for example, speed. The horizontal axis indicates the time passage and the vertical axis indicates the heart rate and the speed in terms of voltages. Assumerthat the mobile unit speed changes from stable state v₁ to stable state v₂ at time to. The mobile unit speed makes the transition from v₁ to v₂ requiring transition time t₁. At this time, the heart rate of the driver changes from n₁ to n₂ requiring transition time t₂. Since the speed is increased, the driver is strained and the heart rate rises.

Usually, considering a vehicle as the mobile unit, the transition time t₂ is long as compared with the transition time t₁. Therefore, if the vehicle is placed in a stable state, the biological state of the driver is transient and the biological information at this time is not that in a steady state. Therefore, if it is not considered that the biological condition of the driver is stabilized in addition to the fact that the vehicle behavior is stabilized, appropriate biological information as vehicle control information cannot be provided. Thus, as the time for which the biological condition is stabilized, time To longer than the transition time t₂ is set as the time to the actual measurement start of the biological condition, and after the expiration of the time To, data is input at sample time tₛ. If data is input two or more times, biological information in time periods tₛ (B), tₛ (C), tₛ (D),... is input in order as the data. In time period tₛ (A), whether or not the biological condition is stabilized is unknown and the biological information in time period is not used as the data.

Next, whether or not the biological information.in the time periods tₛ (B), tₛ(C), tₛ (D),... is to be validated is determined based on whether or not vehicle condition change is within a predetermined range ± ΔV, as shown in FIG. 3. In the figure, in the time periods tₛ (B) and tₛ (C), it is determined that the vehicle condition change exceeds the allowable range ± ΔV, and thus the biological information in the time periods is determined invalid and the biological information in the next time period tₛ (D) is used as the valid information. That is, it is determined that the biological conditions in the time periods tₛ (B) and tₛ (C) are affected by the vehicle behavior change.

Next, an operation flow of the biological state measurement apparatus will be discussed with reference to FIG. 4.

In FIG. 4, first the allowable fluctuation width of the speed of the mobile unit in the stable state of the speed of the mobile unit is set (± ΔV) (step S101). Next, the time for which the behavior condition of the mobile unit is stabilized (t₁) is set (step S102). Next, the time for which the biological condition is stabilized (To) is set (step S103). They may be set at the apparatus manufacturing time or may be set in response to the unique characteristics to the user and the mobile unit and the learning effect after .the user gets the apparatus.

Next, after the speed of the mobile unit is changed, speed (v) is measured to determine the stability of the behavior of the mobile unit (step S104). Next, whether or not the speed (v) is in the allowable fluctuation width range is determined (step S105). If the speed (v) is not in-the range (NO at step S105), it is determined that the mobile unit is not in the stable state, and control returns to step S104 and again the speed (v) is measured. On the other hand, if the speed (v) is in the range (YES at step S105), then whether or not the operation transition time of the mobile unit (t₁) has elapsed is determined (step S106). If t₁ has not yet elapsed (NO at step S106), it is determined that the mobile unit is not in the stable state, and control returns to step S104 and again the speed (v) is measured.

If t₁ has elapsed (YES at step S106), then whether or not the time for which the biological condition of the operator is stabilized (To) has elapsed is determined (step S107). If the time (To) has not yet elapsed (NO at step S107), it is determined that the biological condition is not stabilized, and a wait is made for the time (To) to elapse. If the time (To) has elapsed (YES at step S107), change in the biological condition caused by change in the behavior condition of the mobile unit is measured (step S108).

The biological condition is measured during a predetermined time period of the drive stable time period (step S109) and whether or not the vehicle speed is in the allowable fluctuationwidth (±ΔV) range during the measurement time period is determined (step S110). If the vehicle speed is not in the range (NO at step S110), it is determined that the speed of the mobile unit is changed and therefore that the biological condition of the operator is affected, and the biological information at the time is discarded. Again, control returns to step S104 and the speed (v) is measured. On the other hand, if the speed is in the range (YES at step S110), it is determined that the value is the measurement value of the biological condition when stabilized in the state after the speed of the mobile unit changes, and the biological information is recorded as the data (step S111) and is provided as data for operation control of the vehicle.

As described above, the biological condition measurement apparatus of the invention measures the biological condition in the stable state after behavior operation change of the mobile unit and in the stable state of fluctuation of the biological condition caused by behavior operation change of the mobile unit, so that it is made possible to provide high-accuracy biological information. The biological condition measurement apparatus can be applied to various mobile units.

### (Second embodiment)

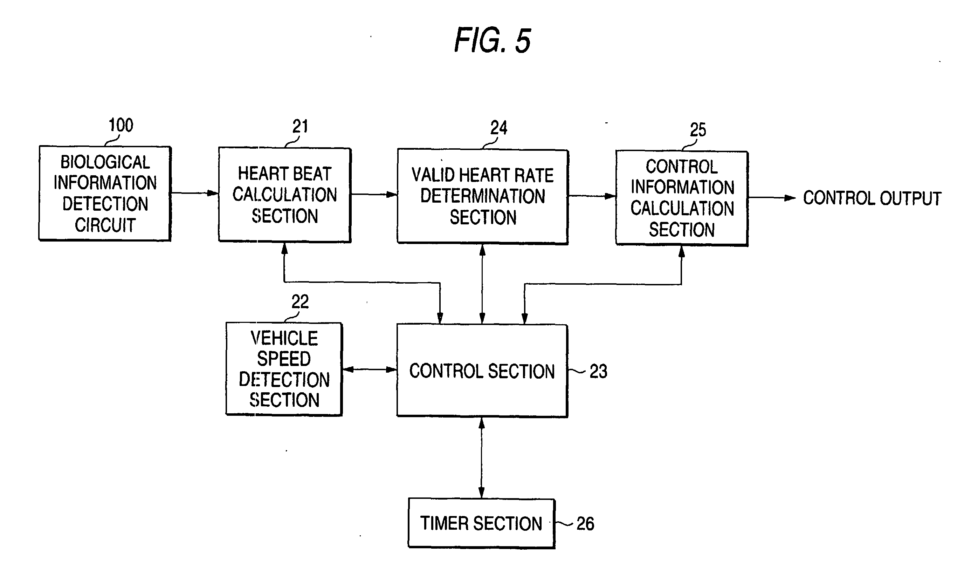

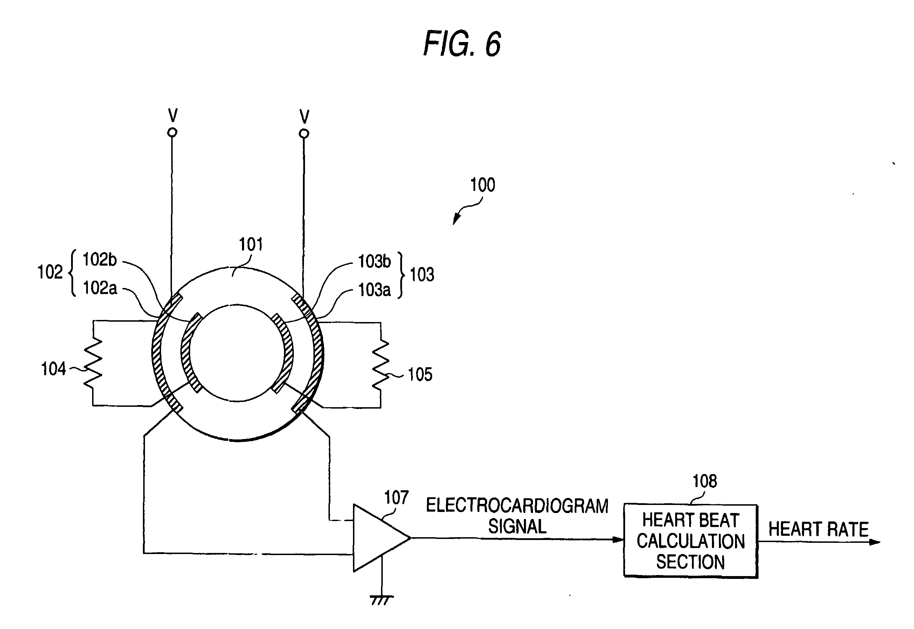

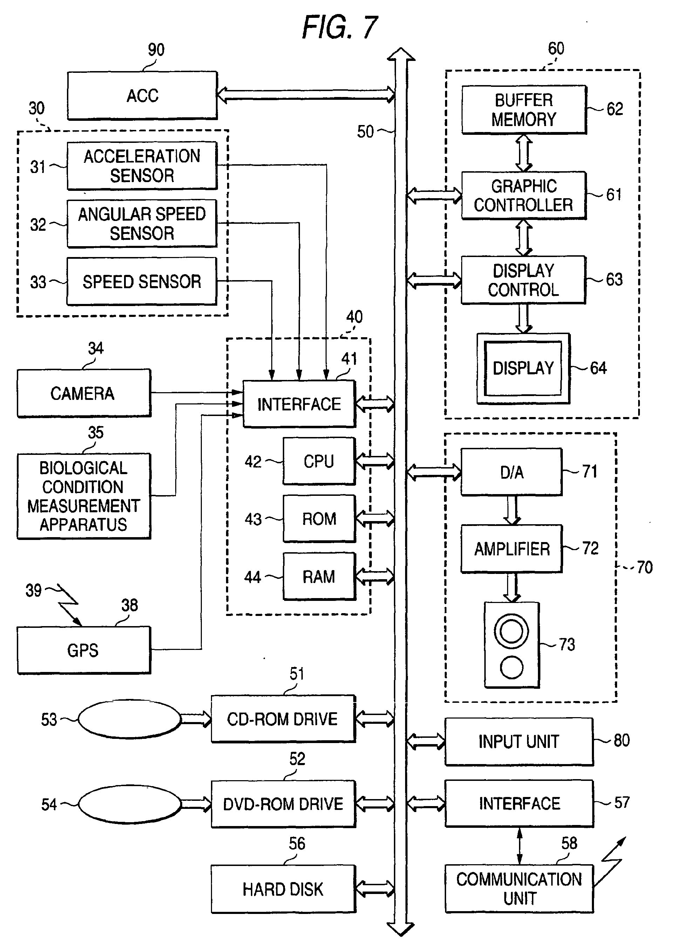

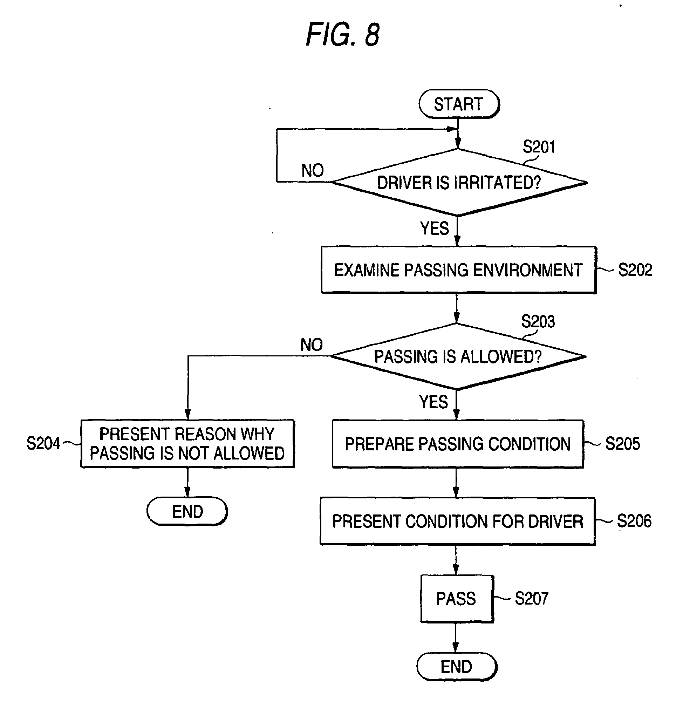

Next, a second embodiment of the invention will be discussed with reference to FIGS. 5 to 8.. The embodiment is a navigation system to which biological information is input wherein a biological condition measurement apparatus of the invention is integral with a vehicle-installed navigation system. FIG. 5 is a block diagram to show the configuration of a biological state measurement apparatus according to a second embodiment of the invention, FIG. 6 is a block diagram to show the configuration of a biological condition sensor section of the biological state measurement apparatus, FIG. 7 is a block diagram to show the configuration of a navigation system incorporating the biological state measurement apparatus, and FIG. 8 is a flowchart to show an operation flow of the navigation system.

To begin with, the configuration of the biological state measurement apparatus used in the embodiment will be discussed with reference to FIGS. 5 and 6.

As shown in FIG. 6, the biological state measurement apparatus comprises a biological information detection circuit 100, a heart rate calculation section 21, a vehicle speed detection section 22, a control section 23, a valid heart rate determination section 24, a control information calculation section 25, and a timer section 26.

The biological information detection circuit 100 is an example of a unit for providing biological information and detects the conduction state, the potential difference, etc., between electrodes when the driver grips a steering wheel

The configuration and the operation of the biological information detection circuit 100 will be discussed with reference to FiG. 6.

In FIG. 6, a steering wheel 101 is provided with electrodes 102a and 102b separated inside and outside and electrodes 103a and 103b separated inside and outside in left and right parts touched by the driver. As the driver grips the parts, left-hand skin resistance 104 is inserted between the electrodes 102a and 102b making up a left-hand electrode 102 and right-hand skin resistance 105 is inserted between the electrodes 103a and 103b making up a right-hand electrode 103. The potentials in the electrodes 102a and 103a are input to a differential amplifier 107, which then amplifies the potential difference between both the electrodes and limits a frequency band, thereby outputting an electrocardiogram signal. This electrocardiogram signal is input to a heart rate calculation section 108. The heart rate is obtained based on the output of the heart rate calculation section 108.

To detect an electrocardiogram, a pulse wave to detect the peripheral blood amount in the hand put on the steering wheel by an optical sensor installed in the steering wheel section may be used or a feeble microwave may be applied to the heart by a high-frequency circuit installed in a seatbelt, etc., and the phase difference between the applied signal and its return signal may be analyzed, thereby measuring the heart rate.

The circuit for detecting the biological information is not limited to the above-described circuit configuration in addition to the heart rate of the driver, sweating, skin resistance, respiration, heart rate variability component, and the like may be to be detected. One or a combination of any two or more items may be used to determine the physical and mental biological conditions of the driver when the driver drives the vehicle.

Referring again to FIG. 5, the heart rate calculation section 21 finds the heart rate from the biological information sent from the biological information detection circuit 100. The heart rate and the vehicle speed are closely related to each other, and the heart rate is data well representing the biological condition of the driver. The vehicle speed detection section 22 supplies speed data as data to determine whether or not the speed is placed in a stable state. If the control section 23 determines that after the vehicle speed is changed, the vehicle runs stably at the speed based on the speed data from the vehicle speed detection section 22, the control section 23 instructs the valid heart rate determination section 24 to send the heart rate from the heart rate calculation section 21 as the valid biological information to input to the control information calculation section 25, which then analyzes the physical or mental biological condition of the driver and outputs vehicle control information based on the analysis result. The biological state measurement apparatus also has the timer section 26 for measuring the time for determining whether or not the vehicle behavior is stable, the time for determining whether or not the biological condition of the driver is stable, the data acquisition time of the biological condition, and the like.

The control information may be presented for the driver from a display or an acoustic device of the navigation system and the actual operation may be left to the driver's determination or the danger avoidance operation at the emergency time may be performed automatically.

Next, the navigation system integral with the biological condition measurement apparatus will be discussed.

As shown in FIG. 7, the navigation system comprises a self-contained position measurement unit 30, a camera 34, the biological condition measurement apparatus 35, a GPS receiver 38, a system controller 40, a CD-ROM drive 51, a DVD-ROM drive 52, a hard disk unit 56, a communication interface 57, a communication unit 58, a display unit 60, an audio output unit 70, and an input unit 80.

The self-contained position measurement unit 30 comprises an acceleration sensor 31, an angular speed sensor 32, and a speed sensor 33. The acceleration sensor 31, which is implemented as a piezoelectric element, for example, detects acceleration of the vehicle and outputs acceleration data. The angular speed sensor 32, which is implemented as an oscillation gyro, for example, detects the angular speed of the vehicle when the vehicle direction is changed, and outputs angular speed data and relative azimuth data. The speed sensor 33 is implemented-as a vehicle speed sensor for detecting rotation of an axel of the vehicle mechanically, magnetically, or optically and generates a vehicle speed pulse of a pulse signal every rotation at a predetermined angle in the axel.

The camera 34 takes a picture of the surrounding of the vehicle, such as the front of the vehicle, for recognizing the circumstances surrounding the vehicle; a CCD camera using a CCD image pickup device is small-sized and is preferred as the camera 34.

The GPS receiver 38 is used to detect the absolute position of the vehicle from latitude and longitude information, etc., and is a reception portion of a radio wave 39 carrying downward line data containing position measurement data from a plurality of GPS satellites. Like the self-contained position measurement unit 30, the GPS receiver 38 is used to know the current position of the vehicle.

The system controller 40 includes an interface 41, a CPU (Central Processing Unit) 42, ROM (Read-Only Memory) 43, and RAM (Random Access Memory) 44 for controlling the whole system

The interface 41 performs interface operation with the acceleration sensor 41, the angular speed sensor 42, the speed sensor 43, the camera 44, and the GPS receiver 48. The vehicle speed data, the acceleration data, the angular speed data, the relative azimuth data, the GPS position measurement data, the absolute azimuth data, the surrounding image data, and the like from the components are input to the system controller 40.

The CPU 42 has the functions of controlling the whole system controller 40 and determining, preparing, and presenting information presented for the driver based on various input data.

The ROM 43 has nonvolatile memory (not shown) storing a control program for controlling the system controller 40 and the like. The RAM 44 stores various data such as route data preset by the user through the input unit 80 in such a manner that the data can be read, and provides a working area for the CPU 42.

The system controller 40, the CD-ROM drive 51, the DVD-ROM drive 52, the hard disk unit 56, the communication interface 57, the display unit 60, the audio output unit 70, and the input unit 80 are connected to each other via a bus line 50.

The CD-ROM drive 51 and the DVD-ROM drive 52 read various data such as road data containing the number of lanes, the road width, the presence or absence of a passing lane, the legal speed, etc., and a control program corresponding to then embodiment from a CD-ROM 53 and a DVD-ROM 54 and output the data under the control of the system controller 40.

Further, the CD-ROM. drive 51 and the DVD-ROM drive 52 may be provided with a function of reading audio data and video data as content information from the CD-ROM 53 and the DVD-ROM 54 under the control of the system controller 40.

The hard disk unit 56 stores, on an irregular or.regular basis, for example, the map data read from the CD-ROM drive 51 or the DVD-ROM drive 52 and corresponding to the position information during running measured by the GPS receiver 38, etc., or stores audio data and video data as content information read from the CD-ROM drive 51 and the DVD-ROM drive 52. Accordingly, it is made possible to read and play back content stored on the CD-ROM 53 or the DVD-ROM 54 while reading the map information stored in the hard disk unit 56, or it is made possible to read the map information stored on the CD-ROM 53 or the DVD-ROM 54 while reading and playing back content information stored in the hard disk unit 56. Video information from the camera 34 can also be stored in the hard disk unit 56 at the same time and can also be read and output to provide assist information indicating that passing is allowed and various pieces of navigation information as described later, as required. Further, road information from a road administrator, received by the communication unit 58 can also be stored in the hard disk unit 56 on an irregular or regular basis and can also be read and output to provide assist information indicating that passing is allowed and various pieces of navigation information as described later, as required

The communication unit 58 is implemented as a mobile telephone, for example, and has a function of downloading audio data, video data, map data, etc., or predetermined type of data concerning the audio data, the video data, the map data, etc., through the communication interface 57 of a modem, etc.

The display unit 60 displays the various display data described above under the control of the system controller 40. The display unit 60 comprises a graphics controller 61 for controlling the whole display unit 60 based on control data sent from the CPU 42 via the bus line 50, buffer memory 62 implemented as memory such as VRAM (Video RAM) for temporarily storing image information that can be displayed immediately, a display control section 63 for performing display control of a display 64 such as a small-sized LCD (Liquid Crystal Display), EL (Electro-Luminescence) display, or a CRT (Cathode Ray Tube) based on image data output from the graphics controller 61, and the display 64. The display 64 is implemented as a liquid crystal display with about 5-to-10-inch diagonal screen, etc., for example, and is mounted in the vicinity of a front panel in the vehicle. The display 64 displays assist information as an image for the driver, determined and prepared by the CPU 42.

The audio output unit 70 comprises a D/A (digital-analog) converter 71 for converting audio digital data sent via the bus line 50 from the CD-ROM drive 51, the DVD-ROM drive 52, the RAM 44, or the like into an audio analog signal, an amplifier (AMP) 72 for amplifying.the audio analog signal output from the D/A converter 71, and a loudspeaker 73 for converting the amplified analog signal into sound for output to the inside of the vehicle under the control of the system controller 40. Assist information by voice for the driver, determined and prepared by the CPU 42 is output through the loudspeaker 73.

The input unit 80 is made up of keys, switches, buttons, a remote control, a voice input unit, etc., for entering various commands and data.

In the embodiment, particularly, the driver operates the input unit 80 to enter a request command for checking the road for congestion or checking whether or not passing is allowed.

Preferably, the input unit 80 is placed in the surrounding of the display 64 installed in the vehicle for convenience of operation.

Next, a flow of the operation of the described navigation system will be discussed with reference to a flowchart of FIG. 8. The following example assumes that the vehicle encounters traffic congestion during running on a road and the driver is confused. Of course, the navigation system of the invention is not limited to the example and can be utilized in various modes.

In FIG. 8, first the biological condition measurement apparatus checks the biological condition of the driver for biological instability to see if the driver is irritated, etc., (step S201). If the driver is not irritated, the routine is repeated. If it is determined that the driver is irritated (YES at step S201), safety of driving is feared and thus whether or not passing operation can be performed in the road environment is examined (step S202). Next, whether or not passing is allowed is determined based on the examination result (step S203).

If the passing operation cannot be performed (NO at step S203), the reason why the passing operation cannot be performed is presented for the driver (step S2004) so as to stabilize the mental condition of the driver.

If it is determined that passing is allowed (YES at step S203), a passing condition is prepared (step S205) and is presented for the driver (step S206). If the driver decides to pass based on the condition, he or she operates the vehicle (step S207).

Further, a computer program is provided for integrating the operation of the navigation system described above and causing the system to function and is read into the CPU 22 from the record medium such as the CD-ROM 53, the DVD-ROM 54, or the hard disk 56 for execution or is downloaded into the CPU 22 through the communication unit 58 for execution.

As an example of the operation environment, in the case where traffic congestion is encountered during running on a road, where the vehicle follows a low-speed vehicle on a mountain path, etc., physical and mental anxiety of the driver increases and the fear of jeopardizing safety occurs. The possibility of passing is automatically examined based on the biological condition of the driver and the possibility of passing including the passing condition can be presented for the driver and it is made possible to secure the safety of driving. If immediate passing is not allowed, information for allowing passing can be presented, so that the driver can be convinced and can be prevented from performing dangerous operation. Further, if passing is not allowed, the reason why passing is not allowed is presented, so that the driver can be convinced and can be made calm.

As described above in detail, the embodiment is the navigation system comprising the navigation apparatus and the biological condition measurement apparatus joined organically for measuring the biological condition of the driver during driving the vehicle and presenting aid in driving and other various services for the driver.

### (Third embodiment)

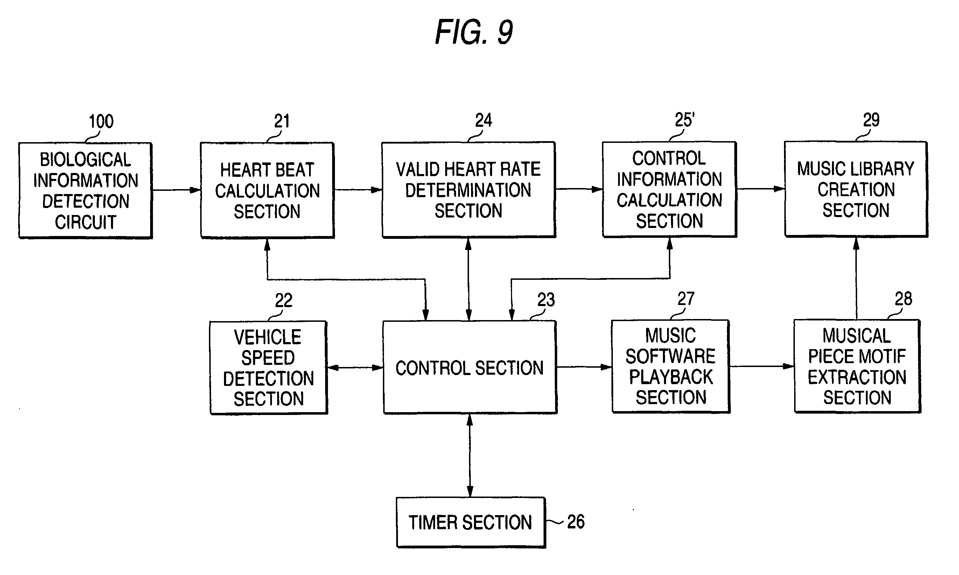

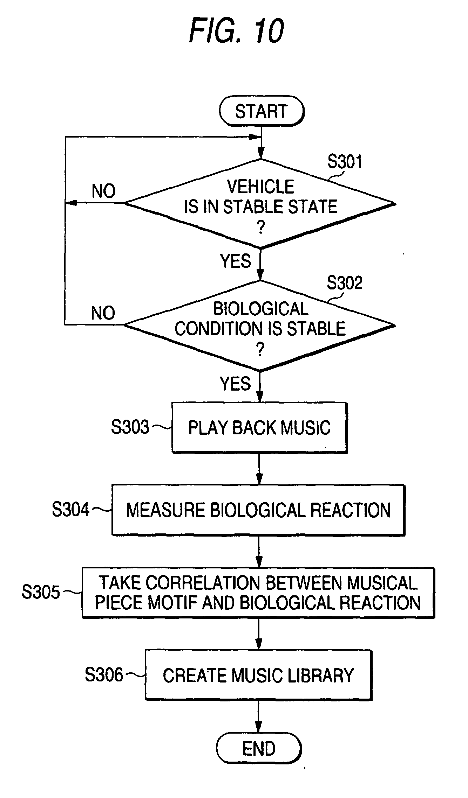

Next, a third embodiment of the invention will be discussed with reference to FIGS. 9 and 10. The embodiment relates to a software library apparatus for creating a software library suiting driver's taste by using the biological condition measurement apparatus in the first embodiment. The software can be.music software and video software. FIG. 9 is a block diagram to show the configuration of the library apparatus according to the third embodiment and FIG. 10 is a flowchart to show an operation flow of the embodiment. Components similar to those previously described with reference to FIG. 5 in the second embodiment are denoted by the same or similar reference numerals in FIG. 9 and will not be discussed again whenever necessary.

As shown in FIG. 9, the library apparatus comprises a biological information detection circuit 100, a heart rate calculation section 21, a vehicle speed detection section 22, a control section 23, a valid heart rate determination section 24, a control information calculation section 25', a timer section 26, a music software playback section 27, a musical piece motif extraction section 28, a music library creation section 29.

The control information calculation section 25' inputs the heart rate from the heart rate calculation section 21 as valid biological information, as instructed from the control section23. Then, it analyzes the physical or mental biological condition of the driver in response to the input biological information and outputs control information for library creation based on the analysis result.

The music software playback section 27 is a player for playing back predetermined software of a CD, etc. It outputs a piece of music through a loudspeaker so that the driver can listen to the piece of music.

The musical piece motif extraction section 28 extracts the musical piece motif of the feature of a played-back musical piece.

The music library creation section 29 takes the correlation between the biological condition of the driver output from the control information calculation section 25' and the musical piece motif extracted by the musical piece motif extraction section 28 and classifies played-back musical pieces based on the correlation, thereby creating a musical piece list, namely, library. More particularly, the music library creation section 29 examines the correlation between the biological condition of the driver indicated by the biological information output from the biological condition measurement apparatus when the biological condition of the driver can be measured precisely and the motif of the musical piece being played back at the time. Thus, when the biological condition is measured precisely, if the driver listens to a musical piece and the biological condition of the driver changes, the vehicle reaches a stable state and thus the change in the biological condition can be regarded as the pure personal biological reaction of the driver to the musical.piece that the driver listens to. Thus, if the correlation between the biological information and the motif of the played-back musical piece is taken, a music software library consisting of the musical pieces having the musical piece motif responsive to the taste of the driver can be created.

For example, if the biological condition when the driver feels enjoyable, that when the driver feels sad, that when the driver feels unpleasant, and the like are ascertained, the musical piece motif when the driver feels enjoyable is determined by correlation analysis and the musical pieces having the musical piece motif are registered with the theme of "enjoyable musical pieces" for the driver, whereby a library can be constructed. Alternatively, the musical piece motif when the driver feels sad is determined and the musical pieces having the musical piece motif are registered with the theme of "sad musical pieces" for the driver, whereby a library can be constructed.

Particularly, as the extracted musical piece motif data is accumulated, it is made possible to construct a library along the driver's taste based on the motif of each musical piece although the musical pieces are not actually played back.

In the embodiment, the musical piece motif is extracted before the correlation between the musical piece motif and the biological condition is taken, but the musical piece motif can also be previously set for or given to each musical piece. For example, the motif of a large number of musical pieces may be input at a stroke from a database of motifs of a large number of musical pieces or the driver may enter the musical piece motif for each musical piece one by one by manual operation, etc.

Further, if the musical piece motif is previously related to a large number of musical pieces, if the correlation between the biological condition and the musical piece motif for a specific driver can be taken by playing back a reasonable number of musical pieces without playing back all musical pieces, it is made possible to construct a library suiting the driver's taste based on the motif of each musical piece.

In the embodiment, the correlation between the musical piece motif and the biological condition is taken, but simply the correlation between each musical piece itself and the biological condition may be taken. That is, in doing so, it is possible to classify music pieces according to the driver's sensibility as to whether each musical piece is an "enjoyable musical piece" or a "sad musical piece," for example, for the driver, so that it is made possible to construct libraries by driver's biological condition (for example, "enjoyable," "sad," etc.,).

Next, the operation flow of the embodiment will be discussed.

In FIG. 10, first, whether or not the vehicle is in a stable state is determined (step S301) . A wait is made until the vehicle becomes a stable state (NO at step S301). If the vehicle becomes a stable state (YES at step S301), then whether or not the biological condition becomes stable is determined (step S302)

At step S302, a wait is made until the biological condition also becomes stable (NO at step S302). If the biological condition becomes stable (YES at step S302), music is played back in the state in which both the vehicle and the biological condition are stable (step S303). The driver is made to listen to the played-back music and the biological reaction at the time is measured (step S304). Next, the motif of the played-back musical piece is extracted and the correlation between the extracted musical piece motif and the measured biological condition is taken (step S305) and music libraries by user's biological condition (for example, "enjoyable," "sad," etc.,) are created based on the correlation (step S306).

In the example shown in FIG. 10, after both the vehicle condition and the biological condition become stable at steps S301 and S302, music is played back at step S303. However, the vehicle condition and the biological condition may be determined at steps S301 and S302 while music is being played back at step S303, and when both the conditions become stable, steps S304 to S306 may be executed.

A library suiting the driver's taste can also be created by a similar procedure, method for content information such as video information and text information in addition to audio information.

In the embodiments described above, various types of processing are executed in the vehicle-installed navigation system; the communication unit 58 in the vehicle-installed navigation- system (see FIG. 7) can also be utilized for communicating with a server of the Internet, etc., to execute the various types of processing. That is, the system can also be constructed as a communication navigation system. In this case, for example, at least some of the components other than the sensors of the biological condition sensor, the mobile unit condition sensor, etc., shown in FIG. 1, 5, or 9 may be constructed on the server of the Internet. For example, output of the biological condition sensor and output of the mobile unit condition sensor may be transmitted to the server through the communication unit 58, a control signal from the server performing the above-described determination processing, analysis processing, creation processing, etc., in response to the sensor outputs may be received by the communication unit 58, and processing corresponding to the control signal may be performed in the vehicle-installed navigation system side. In addition, the navigation function involved in presenting a surrounding map of the present position and route search or route guide, etc., after a destination is input, basic navigation processing can also be executed while communicating with the server using the communication unit 58.

Further, the mobile unit navigation system of the invention can be applied not only to the vehicle-installed navigation system as in the embodiment, but also to navigation systems for various mobile units such as an airplane, a ship, and a bicycle and pedestrians using a portable information terminal, a mobile telephone, etc.

The invention is not limited to the above-described embodiments and can be changed whenever necessary without departing from the scope of the invention read from the claims and the whole specification and a biological condition measurement apparatus and method, a mobile unit navigation system and method, a library apparatus, andacomputer program involving such changes are also contained in the technical idea of the invention.

As described above in detail, according to the biological condition measurement apparatus and method of the invention, whether the provided biological information is biological information based on condition change of the mobile unit or in a new stable state of the mobile unit is determined precisely, and it is made possible to provide biological information with high accuracy, not affected by noise, etc., when the mobile unit is stable. Automatic measurement is conducted at the timing appropriate for measurement and measurement is stopped at other times, so that it is made possible to decrease the CPU load and power consumption involved in processing.

According to a mobile unit navigation system comprising the biological condition measurement apparatus, appropriate mobile unit operation information is provided for the operator and safe and comfortable operation is made possible.

Further, the biological condition measurement apparatus of the invention also enables the operator to easily create a library, etc., of music, movies, etc., suiting taste of the operator.

## Claims

1. A biological condition measurement apparatus (35) comprising:
a behaviour condition detector(12) which detects the behaviour condition of a mobile unit;
a mobile unit stable state determining device (17) which determines whether or not said mobile unit is in a stable state based on the behaviour condition detected by said behaviour condition detector;
a biological condition detector (11) which detects the biological condition of an operator operating said mobile unit;
an operator stable state determining device (17) which determines whether or not the operator is in a stable state based on the biological condition detected by said biological condition detector; and
a processor (15) which adopts biological information indicating the biological condition detected by said biological condition detector as valid biological information, and executes at least one of analysis processing, record processing, and output processing of the biological information, if said mobile unit stable state determining device determines that said mobile unit is in a stable state and said operator stable state determining device determines that the operator is in a stable state
**characterised in that** said processor starts processing of the biological information after the expiration of a predetermined time To since said behaviour condition detector detects a change in the behaviour condition of said mobile unit.

2. A biological condition measurement apparatus (35) according to claim 1, wherein, if fluctuation of the behaviour condition detected by said behaviour condition detector (12) is within a predetermined range, said mobile unit stable state determining device (17) determines that said mobile unit is in a stable state.

3. A biological condition measurement apparatus (35) according to in claim 1, wherein, if fluctuation of the biological condition detected by said biological condition detector (11) is within a predetermined range, said operator stable state determining device (17) determines that the operator is in a stable state.

4. A biological condition measurement apparatus (35) according to claim 1, wherein the predetermined time Tₒ is capable of being set arbitrarily.

5. A biological condition measurement apparatus (35) according to claim 1, wherein said processor (15) performs processing of the biological information at predetermined time tₛ after the expiration of the predetermined time Tₒ.

6. A biological condition measurement apparatus (35) according to claim 5, wherein, when said processor (15) performs processing of the biological information at the predetermined time tₛ,
if said mobile unit stable state determining device (17) determines that the behaviour of said mobile unit within analysis time is in a stable state, said processor validates the biological information; and
if said mobile unit stable state determining device does not determine that the behaviour of said mobile unit is in a stable state, said processor again performs processing of the biological information at the predetermined time tₛ.

7. A biological condition measurement apparatus according to claim 5, wherein the predetermined time tₛ is capable of being set arbitrarily.

8. A biological condition measurement apparatus (35) according to claim 5, wherein:
said processor (15) performs processing of the biological information at two or more successive intervals of the predetermined time tₛ after the expiration of predetermined time Tₒ, and compares the processing results, and
if an error between the processing results is within a predetermined range, validates the biological information.

9. A biological condition measurement apparatus (35) according to claim 1, wherein the biological condition is physical and mental physiological conditions of the operator based on at least one of pulse, sweating, skin resistance, respiration, heart rate, and heart rate variability component.

10. A biological condition measurement method comprising:
a behaviour condition detection step of detecting the behaviour condition of a mobile unit;
a mobile unit stable state determination step of determining whether or not said mobile unit is in a stable state based on the behaviour condition detected in said behaviour condition detection step;
a biological condition detection step of detecting the biological condition of an operator operating said mobile unit;
an operator stable state determination step of determining whether or not the operator is in a stable state based on the biological condition detected in said biological condition detection step; and
a processing step of adopting biological information indicating the biological condition detected in said biological condition detection step as valid biological information, and executing at least one of analysis processing, record processing, and output processing of the biological information, if it is determined in said mobile unit stable state determination step that the mobile unit is in a stable state, and it is determined in said operator stable state determination step that the operator is in a stable state,
**characterised by** starting processing of the biological information after the expiration of a predetermined time To since detecting a change in the behaviour condition of the mobile unit.

11. A mobile unit navigation system comprising:
a biological condition measurement apparatus (35) according to claim 1;
a mobile unit controller(40) which applies control to the run state of said mobile unit based on the processing result of said processor (15);
a current position detector (38) which detects the current position of said mobile unit; and
an information presenter (60,70) which presents guide information for said mobile unit based on the detected current position.

12. A mobile unit navigation system in claim 11, further comprising a communicator (58) which receives at least either of the guide information and source information of the guide information via an external communication network.

13. A mobile unit navigation system according to claim 11, further comprising:
a run environment sensor which senses the run environment of said mobile unit;
a run state sensor which senses the run state of said mobile unit; and
an ACC (Adaptive Cruise Control) unit which controls running of said mobile unit based on information provided for determination of operation of said mobile unit, sensed by said run environment sensor and said run state sensor.

14. A mobile unit navigation method comprising:
a biological condition measurement method according to claim 10;
a mobile unit control step of applying control to the run state of said mobile unit based on the processing result of said processing step;
a current position detection step of detecting the current position of said mobile unit; and
an information presenting step of presenting guide information for said mobile unit based on the detected current position.

15. A mobile unit navigation method according to claim 14, further comprising a communication step of receiving at least either of the guide information and source information of the guide information via an external communication network.

16. A mobile unit navigation method according to claim 14, further comprising:
a run environment sense step of sensing the run environment of said mobile unit;
a run state sense step of sensing the run state of said mobile unit; and
a run control step of an ACC (Adaptive Cruise Control) unit which controls running of said mobile unit based on information provided for determination of operation of said mobile unit, sensed in said run environment sense step and said run state sense step.

17. A library apparatus comprising:
a biological condition measurement apparatus (35) according to claim 1;
an outputting device which outputs content information including at least one of audio information, video information, and text information;
a correlation analyzer (29) which takes the correlation between the biological information processed by said processor (15) and the content information when the content information is output; and
a library creator (29) which creates a library of the content information suiting taste of the operator based on the analysis result of said correlation analyzer.

18. A library apparatus according to claim 17, wherein:
for the content information, feature information indicating the feature in the contents of the content information is preset, and
said correlation analyzer(29) takes the correlation between the preset feature information in place of the content information and the biological information.

19. The library apparatus as claimed in claim 17, further comprising an extractor (28) which extracts features in the contents of the content information from the content information, wherein said correlation analyzer (29) takes the correlation between feature information indicating the feature extracted by said extractor in place of the content information and the biological information.

20. A computer program for causing a computer to function as:
a mobile unit stable state determining circuit (17) which determines whether or not a mobile unit is in a stable state based on the behaviour condition of said mobile unit detected by a behaviour condition detector (12);
an operator stable state determining circuit (17) which determines whether or not an operator operating said mobile unit is in a stable state based on the biological condition of the operator detected by a biological condition detector (11); and
a processor (15) which adopts biological information indicating the biological condition detected by said biological condition detector as valid biological information, and executes at least one of analysis processing, record processing, and output processing of the biological information, if said mobile unit stable state determining circuit determines that said mobile unit is in a stable state and the operator stable state determining circuit determines that the operator is in a stable state
**characterised in that** said program causes said computer, in functioning as said processor, to start processing of the biological information after the expiration of a predetermined time To since said behaviour condition detector detects a change in the behaviour condition of said mobile unit.

## Patentansprüche

1. Körperbefindlichkeitsmesseinrichtung (35), umfassend:
einen Verhaltensbefindlichkeitsdetektor (12), der die Verhaltensbefindlichkeit einer Mobileinheit erfasst;
eine Mobileinheitsstabilzustandsbestimmungsvorrichtung (17), die auf Grundlage der von dem Verhaltensbefindlichkeitsdetektor erfassten Verhaltensbefindlichkeit bestimmt, ob die Mobileinheit in einem stabilen Zustand ist;
einen Körperbefindlichkeitsdetektor (11), der die Körperbefindlichkeit eines die Mobileinheit bedienenden Bedieners erfasst;
eine Bedienerstabilzustandsbestimmungsvorrichtung (17), die auf Grundlage der von dem Körperbefindlichkeitsdetektor erfassten Körperbefindlichkeit bestimmt, ob der Bediener in einem stabilen Zustand ist; und
einen Prozessor (15), der Körperinformation, die die von dem Körperbefindlichkeitsdetektor erfasste Körperbefindlichkeit angibt, als valide Körperinformation übernimmt und wenigstens eine Analyseverarbeitung, eine Aufzeichnungsverarbeitung oder eine Ausgabeverarbeitung der Körperinformation vornimmt, wenn die Mobileinheitsstabilzustandsbestimmungsvorrichtung bestimmt, dass die Mobileinheit in einem stabilen Zustand ist, und die Bedienerstabilzustandsbestimmungsvorrichtung bestimmt, dass der Bediener in einem stabilen Zustand ist;
**dadurch gekennzeichnet, dass** der Prozessor die Verarbeitung der Körperinformation nach Ablauf einer vorbestimmten Zeit T₀ ab der von dem Verhaltensbefindlichkeitsdetektor vorgenommenen Erfassung einer Änderung der Verhaltensbefindlichkeit der Mobileinheit beginnt.

2. Körperbefindlichkeitsmesseinrichtung (35) nach Anspruch 1, bei der die Mobileinheitsstabitzustandsbestimmungsvorrichtung (17) bestimmt, dass die Mobileinheit in einem stabilen Zustand ist, wenn die Schwankung der von dem Verhaltensbefindlichkeitsdetektor (12) erfassten Verhaltensbefindlichkeit innerhalb eines vorbestimmten Bereiches ist.

3. Körperbefindlichkeitsmesseinrichtung (35) nach Anspruch 1, bei der die Bedienerstabilzustandsbestimmungsvorrichtung (17) bestimmt, dass der Bediener in einem stabilen Zustand ist, wenn die Schwankung der von dem Körperbefindlichkeitsdetektor (11) erfassten Körperbefindlichkeit innerhalb eines vorbestimmten Bereiches ist.

4. Körperbefindlichkeitsmesseinrichtung (35) nach Anspruch 1, bei der die vorbestimmte Zeit Tₒ beliebig gewählt werden kann.

5. Körperbefindlichkeitsmesseinrichtung (35) nach Anspruch 1, bei der der Prozessor (15) die Verarbeitung der Körperinformation zu einer vorbestimmten Zeit tₛ nach Ablauf der vorbestimmten Zeit T₀ vornimmt.

6. Körperbefindlichkeitsmesseinrichtung (35) nach Anspruch 5, bei der, wenn der Prozessor (15) eine Verarbeitung der Körperinformation zu der vorbestimmten Zeit tₛ vornimmt:
der Prozessor, wenn die Mobileinheitsstabilzustandsbestimmungsvorrichtung (17) bestimmt, dass das Verhalten der Mobileinheit innerhalb einer Analysezeit in einem stabilen Zustand ist, die Körperinformation validiert; und
der Prozessor, wenn die Mobileinheitsstabilzustandsbestimmungsvorrichtung nicht bestimmt, dass das Verhalten der Mobileinheit in einem stabilen Zustand ist, erneut eine Verarbeitung der Körperinformation zu der vorbestimmten Zeit tₛ vornimmt.

7. Körperbefindlichkeitsmesseinrichtung nach Anspruch 5, bei der die vorbestimmte Zeit tₛ beliebig gewählt werden kann.

8. Körperbefindlichkeitsmesseinrichtung (35) nach Anspruch 5, bei der:
der Prozessor (15) eine Verarbeitung der Körperinformation in zwei oder mehr aufeinanderfolgenden Intervallen der vorbestimmten Zeit tₛ nach Ablauf der vorbestimmten Zeit T₀ vornimmt und die Verarbeitungsergebnisse vergleicht; und
die Körperinformation validiert, wenn ein Fehler bei den Verarbeitungsergebnissen innerhalb eines vorbestimmten Bereiches liegt.

9. Körperbefindlichkeitsmesseinrichtung (35) nach Anspruch 1, bei der die Körperbefindlichkeit die körperliche und geistige physiologische Befindlichkeit des Bedieners auf Grundlage wenigstens einer der Komponenten Puls, Schwitzen, Hautwiderstand, Atmung, Herzschlagrate und Herzschlagratenänderung ist.

10. Körperbefindlichkeitsmessverfahren, umfassend:
einen Verhaltensbefindtichkeitserfassungsschritt des Erfassens der Verhaltensbefindlichkeit einer Mobileinheit;
einen Mobileinheitsstabilzustandsbestimmungsschritt des Bestimmens, ob die Mobileinheit in einem stabilen Zustand ist, auf Grundlage der in dem Verhaltensbefindlichkeitserfassungsschritt erfassten Verhaltensbefindlichkeit;
einen Körperbefindlichkeitserfassungsschritt des Erfassens des Körperbefindlichkeit eines die Mobileinheit bedienenden Bedieners;
einen Bedienerstabilzustandsbestimmungsschritt des Bestimmens, ob der Bediener in einem stabilen Zustand ist, auf Grundlage der in dem Körperbefindlichkeitserfassungsschritt erfassten Körperbefindlichkeit; und
einen Verarbeitungsschritt des Übernehmens von Körperinformation, die die in dem Körperbefindlichkeitserfassungsschritt erfasste Körperbefindlichkeit angibt, als valide Körperinformation und des Vomehmens wenigstens einer Analyseverarbeitung, einer Aufzeichnungsverarbeitung oder einer Ausgabeverarbeitung der Körperinformation, wenn in dem Mobileinheitsstabilzustandsbestimmungsschritt bestimmt wird, dass die Mobileinheit in einem stabilen Zustand ist, und in dem Bedienerstabilzustandsbestimmungsschritt bestimmt wird, dass der Bediener in einem stabilen Zustand ist;
**dadurch gekennzeichnet, dass** die Verarbeitung der Körperinformation nach Ablauf einer vorbestimmten Zeit Tₒ ab der Erfassung einer Änderung der Verhaltensbefindlichkeit der Mobileinheit beginnt.

11. Mobileinheitsnavigationssystem, umfassend:
eine Körperbefindlichkeitsmesseinrichtung (35) nach Anspruch 1;
eine Mobileinheitssteuerung (40), die eine Steuerung an dem Laufzustand der Mobileinheit auf Grundlage des Verarbeitungsergebnisses des Prozessors (15) vornimmt;
einen Momentanpositionsdetektor (38), der die Momentanposition der Mobileinheit erfasst; und
einen Informationsdarbieter (60, 70), der Leitinformation für die Mobileinheit auf Grundlage der erfassten Momentanposition darbietet.

12. Mobileinheitsnavigationssystem nach Anspruch 11, des Weiteren umfassend einen Kommunikator (58), der wenigstens entweder die Leitinformation oder die Quelleninformation der Leitinformation über ein externes Kommunikationsnetzwerk empfängt.

13. Mobileinheitsnavigationssystem nach Anspruch 11, des Weiteren umfassend:
einen Laufumgebungssensor, der die Laufumgebung der Mobileinheit erfasst;
einen Laufzustandssensor, der den Laufzustand der Mobileinheit erfasst; und
eine ACC-Einheit (Adaptive Cruise Control), die das Laufen der Mobileinheit auf Grundlage von für die Bestimmung des Betriebes der Mobileinheit vorgesehener und von, dem Laufumgebungssensor und dem Laufzustandssensor erfasster Information steuert.

14. Mobileinheitsnavigationsverfahren, umfassend:
ein Körperbefindlichkeitsmessverfahren nach Anspruch 10;
einen Mobileinheitssteuerschritt des Vornehmens einer Steuerung an dem Laufzustand der Mobileinheit auf Grundlage des Verarbeitungsergebnisses des Verarbeitungsschrittes;
einen Momentanpositionserfassungsschritt des Erfassens der Momentanposition der Mobileinheit; und
einen Informationsdarbietungsschritt des Darbietens von Leitinformation für die Mobileinheit auf Grundlage der erfassten Momentanposition.

15. Mobileinheitsnavigationsverfahren nach Anspruch 14, des Weiteren umfassend einen Kommunikationsschritt des Empfangens wenigstens entweder der Leitinformation oder der Quelleninformation der Leitinformation über ein externes Kommunikationsnetzwerk.

16. Mobileinheitsnavigationsverfahren nach Anspruch 14, des Weiteren umfassend:
einen Laufumgebungserfassungsschritt des Erfassens der Laufumgebung der Mobileinheit;
einen Laufzustandserfassungsschritt des Erfassens des Laufzustandes der Mobileinheit; und
einen Laufsteuerschritt einer ACC-Einheit (Adaptive Cruise Control), der das Laufen der Mobileinheit auf Grundlage von für die Bestimmung des Betriebes der Mobileinheit vorgesehener und in dem Laufumgebungserfassungsschritt und dem Laufzustandserfassungsschritt erfasster Information steuert.

17. Bibliothekseinrichtung, umfassend:
eine Körperbefindlichkeitsmesseinrichtung (35) nach Anspruch 1;
eine Ausgabevorrichtung, die wenigstens eine eine Audioinformation, eine Videoinformation und eine Textinformation beinhaltende Inhaltsinformation ausgibt;
einen Korrelationsanalysator (29), der die Korrelation zwischen der von dem Prozessor (15) verarbeiteten Körperinformation und der Inhaltsinformation vornimmt, wenn die Inhaltsinformation ausgegeben wird; und
einen Bibliothekserzeuger (29), der eine Bibliothek der Inhaltsinformation in Abstimmung auf Vorlieben des Bedieners auf Grundlage des Analyseergebnisses des Korrelationsanalysators erzeugt.

18. Bibliothekseinrichtung nach Anspruch 17, bei der:
für die Inhaltsinformation eine das Merkmal in den Inhalten der Inhaltsinformation angebende Merkmalsinformation voreingestellt ist; und
der Korrelationsanalysator (29) die Korrelation zwischen der voreingestellten Merkmalsinformation anstelle der Inhaltsinformation und der Körperinformation vornimmt.

19. Bibliothekseinrichtung nach Anspruch 17, des Weiteren umfassend einen Extraktor (28), der Merkmale in den Inhalten der Inhaltsinformation aus der Inhaltsinformation extrahiert, wobei der Korrelationsanalysator (29) die Korrelation zwischen der das Merkmal gemäß Extraktion durch den Extraktor angebenden Merkmalsinformation anstelle der Inhaltsinformation und der Körperinformation vornimmt.

20. Computerprogramm, das einen Computer in die Lage versetzt, zu arbeiten als:
Mobileinheitsstabilzustandsbestimmungsschaltung (17), die auf Grundlage der von einem Verhaltensbefindlichkeitsdetektor (12) erfassten Verhaltensbefindlichkeit der Mobileinheit bestimmt, ob eine Mobileinheit in einem stabilen Zustand ist,
Bedienerstabilzustandsbestimmungsschaltung (17), die auf Grundlage der von einem Körperbefindlichkeitsdetektor (12) erfassten Körperbefindlichkeit eines Bedieners bestimmt, ob der die Mobileinheit bedienende Bediener in einem stabilen Zustand ist; und
Prozessor (15), der Körperinformation, die die von dem Körperbefindlichkeitsdetektor erfasste Körperbefindlichkeit angibt, als valide Körperinformation übemimmt und wenigstens eine Analyseverarbeitung, eine Aufzeichnungsverarbeitung oder eine Ausgabeverarbeitung der Körperinformation vornimmt, wenn die Mobileinheitsstabilzustandsbestimmungsschaltung bestimmt, dass die Mobileinheit in einem stabilen Zustand ist, und die Bedienerstabilzustandsbestimmungsschaltung bestimmt, dass der Bediener in einem stabilen Zustand ist;
**dadurch gekennzeichnet, dass** das Programm den Computer beim Arbeiten als Prozessor veranlasst, die Verarbeitung der Körperinformation nach Ablauf einer vorbestimmten Zeit T₀ ab der von dem Verhaltensbefindlichkeitsdetektor vorgenommenen Erfassung einer Änderung der Verhaltensbefindlichkeit der Mobileinheit zu beginnen.

## Revendications

1. Appareil de mesure de condition biologique (35), comprenant
un détecteur de condition de comportement (12) qui détecte la condition de comportement d'une unité mobile ;
un dispositif de détermination d'état stable d'unité mobile (17) qui détermine si ladite unité mobile se trouve oui ou non dans un état stable, sur la base de la condition de comportement détectée par ledit détecteur de condition de comportement ;
un détecteur de condition biologique (11) qui détecte la condition biologique d'un opérateur actionnant ladite unité mobile ;
un dispositif de détermination d'état stable d'opérateur (17) qui détermine si l'opérateur se trouve oui ou non dans un état stable, sur la base de la condition biologique détectée par ledit détecteur de condition biologique ; et
un processeur (15) qui adopte des informations biologiques indiquant la condition biologique détectée par ledit détecteur de condition biologique comme des informations biologiques valables, et exécute au moins l'un quelconque d'un traitement d'analyse, d'un traitement d'enregistrement et d'un traitement de sortie des informations biologiques, si ledit dispositif de détermination d'état stable d'unité mobile détermine que ladite unité mobile se trouve dans un état stable et ledit dispositif de détermination d'état stable d'opérateur détermine que l'opérateur se trouve dans un état stable ;
**caractérisé en ce que** ledit processeur commence le traitement des informations biologiques après l'expiration d'un temps prédéterminé T₀ depuis que ledit détecteur de condition de comportement a détecté un changement de la condition de comportement de ladite unité mobile.

2. Appareil de mesure de condition biologique (35) selon la revendication 1, dans lequel, si la variation de la condition de comportement détectée par ledit détecteur de condition de comportement (12) se trouve dans une plage prédéterminée, ledit dispositif de détermination d'état stable d'unité mobile (17) détermine que ladite unité mobile se trouve dans un état stable.

3. Appareil de mesure de condition biologique (35) selon la revendication 1, dans lequel, si la variation de la condition biologique détectée par ledit détecteur de condition biologique (11) se trouve dans une plage prédéterminée, ledit dispositif de détermination d'état stable d'opérateur (17) détermine que l'opérateur se trouve dans un état stable.

4. Appareil de mesure de condition biologique (35) selon la revendication 1, dans lequel le temps prédéterminé T₀ peut être réglé de façon arbitraire.

5. Appareil de mesure de condition biologique (35) selon la revendication 1, dans lequel ledit processeur (15) effectue un traitement des informations biologiques à un temps prédéterminé tₛ après l'expiration du temps prédéterminé T₀.

6. Appareil de mesure de condition biologique (35) selon la revendication 5, dans lequel ledit processeur (15) effectue un traitement des informations biologiques au temps prédéterminé tₛ :
si ledit dispositif de détermination d'état stable d'unité mobile (17) détermine que le comportement de ladite unité mobile à l'intérieur du temps d'analyse se trouve dans un état stable, ledit processeur valide les informations biologiques ; et
si ledit dispositif de détermination d'état stable d'unité mobile ne détermine pas que le comportement de ladite unité mobile se trouve dans un état stable, ledit processeur effectue encore une fois le traitement des informations biologiques au temps prédéterminé tₛ.

7. Appareil de mesure de condition biologique selon la revendication 5, dans lequel le temps prédéterminé tₛ peut être réglé de façon arbitraire.

8. Appareil de mesure de condition biologique (35) selon la revendication 5, dans lequel :
ledit processeur (15) effectue le traitement des informations biologiques à deux ou plusieurs intervalles successifs du temps prédéterminé tₛ après l'expiration du temps prédéterminé T₀, et compare les résultats de traitement ; et
si une erreur entre les résultats de traitement se trouve dans une plage prédéterminée, il valide les informations biologiques.

9. Appareil de mesure de condition biologique (35) selon la revendication 1, dans lequel la condition biologique correspond à des conditions physiques et physiologiques mentales de l'opérateur sur la base d'au moins un élément parmi le pouls, la transpiration, la résistance de la peau, la respiration, la fréquence cardiaque et la composante de variabilité de fréquence cardiaque.

10. Procédé de mesure de condition biologique, comprenant :
une étape de détection de condition de comportement consistant à détecter la condition de comportement d'une unité mobile ;
une étape de détermination d'état stable d'unité mobile consistant à déterminer si ladite unité mobile se trouve oui ou non dans un état stable, sur la base de la condition de comportement détectée dans ladite étape de détection de condition de comportement ;
une étape de détection de condition biologique consistant à détecter la condition biologique d'un opérateur actionnant ladite unité mobile ;
une étape de détermination d'état stable d'opérateur consistant à déterminer si l'opérateur se trouve oui ou non dans un état stable, sur la base de la condition biologique détectée dans ladite étape de détection de condition biologique ; et
une étape de traitement consistant à adopter des informations biologiques indiquant la condition biologique détectée dans ladite étape de détection de condition biologique comme des informations biologiques valables, et à exécuter au moins l'un quelconque d'un traitement d'analyse, d'un traitement d'enregistrement et d'un traitement de sortie des informations biologiques, s'il est déterminé dans ladite étape de détermination d'état stable d'unité mobile que l'unité mobile se trouve dans un état stable, et il est déterminé dans ladite étape de détermination d'état stable d'opérateur que l'opérateur se trouve dans un état stable,
**caractérisé par** le fait de commencer le traitement des informations biologiques après l'expiration d'un temps prédéterminé T₀ depuis la détection d'un changement de la condition de comportement de l'unité mobile.

11. Système de navigation d'unité mobile, comprenant :
un appareil de mesure de condition biologique (35) selon la revendication 1 ;
un contrôleur d'unité mobile (40) qui applique une commande à l'état de déplacement de ladite unité mobile, sur la base du résultat de traitement dudit processeur (15) ;
un détecteur de position actuelle (38) qui détecte la position actuelle de ladite unité mobile ; et
un dispositif de présentation d'informations (60, 70) qui présente des informations de guidage pour ladite unité mobile, sur la base de la position actuelle détectée.

12. Système de navigation d'unité mobile selon la revendication 11, comprenant en outre un dispositif de communication (58) qui reçoit au moins soit les informations de guidage soit des informations source des informations de guidage par l'intermédiaire d'un réseau de communication externe.

13. Système de navigation d'unité mobile selon la revendication 11, comprenant en outre :
un capteur d'environnement de déplacement qui capte l'environnement de déplacement de ladite unité mobile ;
un capteur d'état de déplacement qui capte l'état de déplacement de ladite unité mobile ; et
une unité ACC (système adaptatif de régulation de vitesse) qui commande le déplacement de ladite unité mobile, sur la base d'informations fournies pour la détermination du fonctionnement de ladite unité mobile, captées par ledit capteur d'environnement de déplacement et ledit capteur d'état de déplacement.

14. Procédé de navigation d'unité mobile, comprenant :
un procédé de mesure de condition biologique selon la revendication 10 ;
une étape de commande d'unité mobile consistant à appliquer une commande à l'état de déplacement de ladite unité mobile sur la base du résultat de traitement de ladite étape de traitement ;
une étape de détection de position actuelle consistant à détecter la position actuelle de ladite unité mobile ; et
une étape de présentation d'informations consistant à présenter des informations de guidage pour ladite unité mobile sur la base de la position actuelle détectée.

15. Procédé de navigation d'unité mobile selon la revendication 14, comprenant en outre une étape de communication consistant à recevoir au moins soit les informations de guidage soit des informations de source des informations de guidage par l'intermédiaire d'un réseau de communication externe.

16. Procédé de navigation d'unité mobile selon la revendication 14, comprenant en outre :
une étape de capture d'environnement de déplacement consistant à capter l'environnement de déplacement de ladite unité mobile ;
une étape de capture d'état de déplacement consistant à capter l'état de déplacement de ladite unité mobile ; et
une étape de commande de déplacement d'une unité ACC (système adaptatif de régulation de vitesse) qui commande le déplacement de ladite unité mobile, sur la base des informations fournies pour la détermination du fonctionnement de ladite unité mobile, captées dans ladite étape de capture d'environnement de déplacement et ladite étape de capture d'état de déplacement.

17. Appareil de bibliothèque, comprenant :
un appareil de mesure de condition biologique (35) selon la revendication 1 ;
un dispositif de sortie qui délivre des informations de contenu comprenant au moins l'une quelconque des informations audio, des informations vidéo et des informations de texte ;
un analyseur de corrélation (29) qui établit la corrélation entre les informations biologiques traitées par ledit processeur (15) et les informations de contenu lorsque les informations de contenu sont délivrées ; et
un dispositif de création de bibliothèque (29) qui crée une bibliothèque des informations de contenu à la convenance de l'opérateur, sur la base du résultat d'analyse dudit analyseur de corrélation.

18. Appareil de bibliothèque selon la revendication 17, dans lequel :
pour les informations de contenu, des informations de particularité indiquant la particularité du contenu des informations de contenu sont préréglées ; et
ledit analyseur de corrélation (29) établit la corrélation entre les informations de particularité préréglées au lieu des informations de contenu et des informations biologiques.

19. Appareil de bibliothèque selon la revendication 17, comprenant en outre un extracteur (28) qui extrait des particularités dans le contenu des informations de contenu à partir des informations de contenu, dans lequel ledit analyseur de corrélation (29) établit la corrélation entre des informations de particularité indiquant la particularité extraite par ledit extracteur au lieu des informations de contenu et des informations biologiques.

20. Programme informatique permettant d'amener un ordinateur à fonctionner comme :
un circuit de détermination d'état stable d'unité mobile (17) qui détermine si une unité mobile se trouve oui ou non dans un état stable, sur la base de la condition de comportement de ladite unité mobile détectée par un détecteur de condition de comportement (12) ;
un circuit de détermination d'état stable d'opérateur (17) qui détermine si un opérateur actionnant ladite unité mobile se trouve oui ou non dans un état stable, sur la base de la condition biologique de l'opérateur détectée par un détecteur de condition biologique (11) ; et
un processeur (15) qui adopte des informations biologiques indiquant la condition biologique détectée par ledit détecteur de condition biologique indiquant la condition biologique détectée par ledit détecteur de condition biologique comme des informations biologiques valables, et exécute au moins l'un quelconque d'un traitement d'analyse, d'un traitement d'enregistrement et d'un traitement de sortie des informations biologiques, si ledit circuit de détermination d'état stable d'unité mobile détermine que ladite unité mobile se trouve dans un état stable et que le circuit de détermination d'état stable d'opérateur détermine que l'opérateur se trouve dans un état stable,
**caractérisé en ce que** ledit programme amène ledit ordinateur, en fonctionnant comme ledit processeur, à commencer le traitement des informations biologiques après l'expiration d'un temps prédéterminé T₀ depuis que ledit détecteur de condition de comportement a détecté un changement de la condition de comportement de ladite unité mobile.
